# EUROPEAN PATENT SPECIFICATION

(11) **EP 4 453 568 B1**
(45) Date of publication and mention of the grant of the patent: **28.01.2026**
(21) Application number: 22854781.6
(22) Date of filing: 19.12.2022
(51) Int. Cl.: G01N 33/53, G01N 33/569, G01N 33/68

(54) **METHODS FOR DETERMINING THE PRESENCE AND/OR AMOUNT OF A HUMAN IGG3 ANTIBODY SPECIFIC FOR A FLAVIVIRUS ANTIGEN IN A SAMPLE**
VERFAHREN ZUR BESTIMMUNG DER ANWESENHEIT UND/ODER MENGE EINES MENSCHLICHEN IGG3-ANTIKÖRPERS SPEZIFISCH FÜR EIN FLAVIVIRUSANTIGEN IN EINER PROBE
PROCÉDÉS POUR DÉTERMINER LA PRÉSENCE ET/OU LA QUANTITÉ D'UN ANTICORPS HUMAIN IGG3 SPÉCIFIQUE D'UN ANTIGÈNE DU FLAVIVIRUS DANS UN ÉCHANTILLON

(30) Priority: 21.12.2021 EP 21216535
(43) Date of publication of application: 30.10.2024
(73) Proprietor: Takeda Vaccines, Inc., Cambridge, MA 02139 (US)
(72) Inventor: NASCIMENTO, Eduardo, Cambridge, Massachusetts 02139 (US)
(74) Representative: Maiwald GmbH
(86) International application number: PCT/US2022/081970
(87) International publication number: WO 2023/122556

(56) References cited:
- WO-A1-2016/040320
- WO-A2-2011/046623
- RODRIGUEZ-BARRAQUER ISABEL ET AL: "Impact of preexisting dengue immunity on Zika virus emergence in a dengue endemic region", vol. 363, no. 6427, 8 February 2019 (2019-02-08), US, pages 607 - 610, XP055925783, ISSN: 0036-8075, Retrieved from the Internet <URL:https://www.arca.fiocruz.br/bitstream/icict/36157/2/Impact%20of%20preexisting%20dengue%20immunity%20on%20Zika%20virus%20emergence%20in%20a%20dengue%20endemic%20region..pdf> DOI: 10.1126/science.aav6618
- RODRIGUEZ-BARRAQUER ISABEL ET AL: "Supplementary Materials for Impact of preexisting dengue immunity on Zika virus emergence in a dengue endemic region", SCIENCE, 8 February 2019 (2019-02-08), pages 1 - 32, XP055927136, Retrieved from the Internet <URL:https://www.science.org/doi/suppl/10.1126/science.aav6618/suppl_file/aav6618_rodriguez-barraquer_sm.pdf> [retrieved on 20220601], DOI: 10.1126/science.aav6618
- GRAY ELIN S. ET AL: "Broad Neutralization of Human Immunodeficiency Virus Type 1 Mediated by Plasma Antibodies against the gp41 Membrane Proximal External Region", vol. 83, no. 21, 1 November 2009 (2009-11-01), US, pages 11265 - 11274, XP055925678, ISSN: 0022-538X, Retrieved from the Internet <URL:https://www.ncbi.nlm.nih.gov/pmc/articles/PMC2772769/pdf/1359-09.pdf> DOI: 10.1128/JVI.01359-09
- SCHARF O ET AL: "Immunoglobulin G3 from polyclonal human immunodeficiency virus (HIV) immune globulin is more potent than other subclasses in neutralizing HIV type 1", JOURNAL OF VIROLOGY, THE AMERICAN SOCIETY FOR MICROBIOLOGY, US, vol. 75, no. 14, 1 July 2001 (2001-07-01), pages 6558 - 6565, XP002219983, ISSN: 0022-538X, DOI: 10.1128/JVI.75.14.6558-6565.2001
- ANONYMOUS: "NAb(TM) Spin Kits, 0.2mL for Antibody Purification", 9 April 2013 (2013-04-09), pages 1 - 3, XP055926978, Retrieved from the Internet <URL:https://www.thermofisher.com/document-connect/document-connect.html?url=https://assets.thermofisher.com/TFS-Assets%2FLSG%2Fmanuals%2FMAN0011590_NAb_Spin_0.2mL_Antibody_Purifi_UG.pdf> [retrieved on 20220601]

## Description

### Technical Field of the Invention

The present invention relates to methods for determining the presence and/or amount of a human IgG3 antibody, preferably specific for a virus antigen in a sample. The present invention also relates to the use of the methods in the quality control of viral vaccines, and in the diagnosis of viral infections.

### Background of the Invention

Antibody responses to viral infections in humans are varied and of widely divergent clinical significance. Preexisting, reactive antibodies or antibodies that are formed early during infection can bind to virus particles, forming immune complexes that can neutralize or mediate clearance of the virus. On the other hand, immune complexes can also promote inflammation and exacerbate symptoms of disease. How antibodies within immune complexes modulate infection depends, in part, on their Fc domain structure. Fc structure, in turn, dictates interactions with Fcγ receptors (FcγRs), which are expressed by a variety of cells that are activated during infection.

Antibody isotypes IgG, IgA and IgM are a primary determinant of Fc structure and thus of activity. Initial B cell responses are characterized by the production of IgM antibodies. Production of class-switched IgA and IgG antibodies follows, with IgA playing a central role in mucosal immunity, while IgG is the dominant isotype involved in systemic antiviral immunity. IgG functions are governed by interactions between immune complexes and effector immune cells that express FcγRs, the receptors for IgG. The balance of FcγRs that are engaged by immune complexes determines the degree of the inflammatory effector cell response. Activating, low-affinity FcγRs (FcγRIIa and FcγRIIIa) transduce inflammatory signaling through immunoreceptor tyrosine-based activation motifs (ITAMs); in health, ITAM signaling is balanced by immunoreceptor tyrosine-based inhibition motif (ITIM) signaling through the inhibitory FcγRIIb.

The strength of interactions between immune complexes and various FcγRs is determined by structural diversity within IgG subclasses (IgG1, IgG2, IgG3 and IgG4) and posttranslational modifications of their Fc domains. Importantly, individuals produce distinct structural repertoires of IgG Fc domains, with some producing highly activating/proinflammatory repertoires enriched for features such as IgG1, IgG3 and/or reduced core fucosylation of the IgG1 Fc domain. Others produce IgG repertoires characterized by higher levels of IgG2 and/or sialylated Fcs that have reduced activating/inflammatory FcγR signaling potential (Chakraborty et al., Nature Immunol. 22 (2021), 67-73).

Nascimento et al., J. Virol. Methods 257 (2018), 62-68 describes that anti-dengue NS1-specific IgG and IgG3 are potential biomarkers of long-term and recent (less than 6 months) dengue virus infections, respectively. IgG3 was also used as a marker of recent infection by HIV (Viana et al., Epidemiology & Infection 146 (2018), 1293-1300. Rodriguez-Barraquer et al., Science 363 (6427), 607-610 describe the measurement of the IgG3 response against Zikavirus NS1 protein.

Trend et al., Front. Immunol. 9 (2018), Article 1590: 1-13 outlines that higher serum IgG3 levels may predict the development of Multiple sclerosis in individuals with clinically isolated syndrome.

Chakraborty et al., *supra,* describes that IgG3 and IgG1 with F0N0 glycoform modification are elevated in more patients with severe COVID-19.

There is a need for the provision of an improved assay for determining IgG3 subtype antibodies. In particular, an assay shall be provided wherein the sensitivity and/or specificity is increased over prior art assays for determining IgG3, in particular human IgG3 specific for a virus antigen.

### Summary of the Invention

In a first aspect, the present invention provides a method for determining the presence and/or amount of a human IgG3 antibody specific for a flavivirus antigen in a sample comprising the steps of:
**Step 1:** contacting an amount of the sample with Protein A coupled to beads to allow binding of human IgG1, human IgG2, human IgG4, human IgA, human IgE and human IgM antibodies to the Protein A coupled to the beads;
**Step 2:** separating the human IgG1, human IgG2, human IgG4, human IgA, human IgE and human IgM antibodies bound to the Protein A coupled to the beads from the remaining sample and thereby producing a human IgG3 antibody enriched supernatant;
**Step 3:** removing the human IgG3 antibody enriched supernatant from the human IgG1, human IgG2, human IgG4, human IgA, human IgE and human IgM antibodies bound to the Protein A coupled to the beads;
**Step 4:** contacting a flavivirus antigen with the human IgG3 antibody enriched supernatant to allow binding of the human IgG3 antibody to the flavivirus antigen;
**Step 5:** contacting the human IgG3 antibody bound to the flavivirus antigen with a primary IgG specific antibody or a primary IgG3 specific antibody to allow binding of the primary IgG specific antibody or the primary IgG3 specific antibody to the human IgG3 antibody bound to the flavivirus antigen;
**Step 6:** contacting the bound primary IgG3 specific antibody or the bound primary IgG specific antibody of step 4 with a labelled secondary antibody being specific for the primary IgG3 specific antibody or the primary IgG specific antibody to allow binding of the labelled secondary antibody to the primary antibody; and
**Step 7:** detecting a signal from the labelled secondary antibody bound to the primary antibody in step 5, wherein the signal is indicative for the presence and/or amount of the labelled secondary antibody and wherein the presence and/or amount of the labelled secondary antibody is indicative for the presence and/or amount of human IgG3 antibody specific for a flavivirus antigen in the sample; or wherein Steps 5 to 7 are replaced by:
   **Step 5':** contacting the human IgG3 antibody bound to the flavivirus antigen with a labelled primary IgG specific antibody or a labelled primary IgG3 specific antibody to allow binding of the labelled primary IgG specific antibody or the labelled primary IgG3 specific antibody to the human IgG3 antibody bound to the flavivirus antigen; and
   **Step 6':** detecting a signal from the labelled primary IgG specific antibody or a labelled primary IgG3 specific antibody bound to the flavivirus antigen in step 5', wherein the signal is indicative for the presence and/or amount of the labelled primary IgG specific antibody or a labelled primary IgG3 specific antibody and wherein the presence and/or amount of the labelled primary antibody is indicative for the presence and/or amount of human IgG3 antibody specific for a flavivirus antigen in the sample.

In a second aspect the present invention provides a method for increasing the sensitivity of a method for determining the presence and/or amount of the human IgG3 antibody specific for a flavivirus antigen in a sample comprising performing a step of pre-treatment of the sample containing human IgG3 specific for a flavivirus antigen and one or more of human IgG1, human IgG2, human IgG4, human IgA and human IgM antibodies with immobilized or immobilizable Protein.

In a third aspect, the present invention provides a method for the *in vitro* diagnosis of a flavivirus infection in a human subject within the last six months, preferably a dengue virus or a Zika virus infection, comprising performing the method according to the invention.

In a fourth aspect, the present invention provides a method for determining the human IgG3 response to a flaviviral vaccine, preferably a dengue or Zika vaccine comprising performing the method according to the invention.

The prior art methods for determining IgG3 subtype antibodies such as ELISAs or immunofluorescent assays are based on the use of an IgG3 specific primary antibody. The proportion of IgG3 in biological samples is relatively low, generally below 5 %. In contrast, the proportion of IgG1 and IgG2 is 60 % and 32 %, respectively; see Vidarsson et al., Front. Immunol. 5 (2014), Article 520: 1-17. The present inventors have found that due to the co-presence of high amounts of IgG1 and IgG2 the sensitivity/specificity of the prior art assays is negatively affected. The present inventors have found that by a prior step of removing of human IgM, human IgA, human IgG1, human IgG2 and human IgG4 by the use of Protein A the sensitivity of the detection of human IgG3 in a sample can be significantly increased.

### BRIEF DESCRIPTION OF THE DRAWINGS

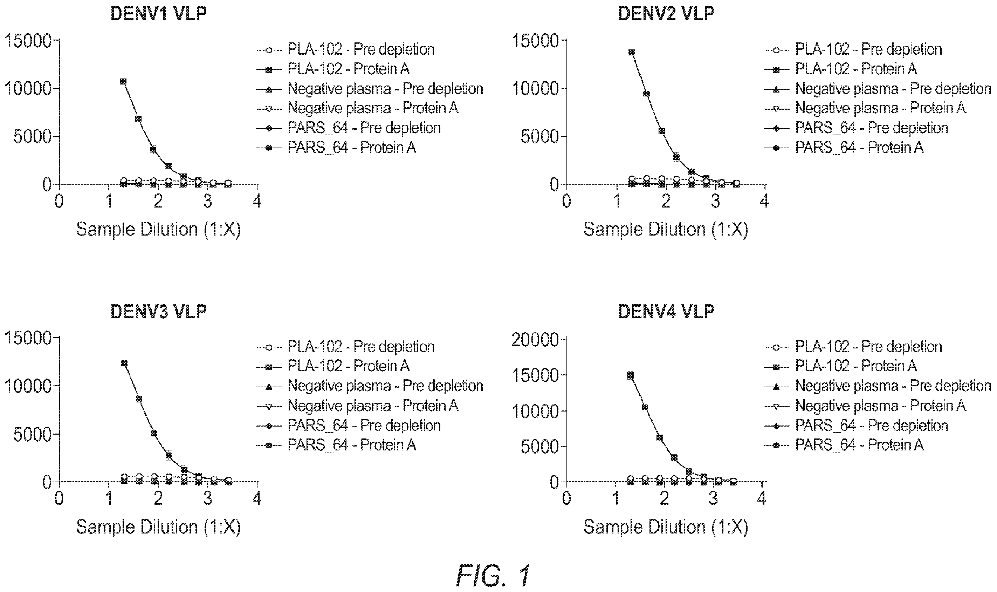

**Figure 1** Determination of the IgG3 amount in different patient samples with the method according to the present invention (designated as Protein A) and a method which did not include the Protein A depletion step before the actual determination of IgG3 (designated as pre-depletion). DENV1 VLP (upper left panel), DENV2 VLP (upper right panel), DENV3 VLP (lower left panel) and DENV4 VLP (lower right panel), respectively, have been used as antigen. Presumed Dengue samples used in Figures 1 to 6: PLA-102, PLA-116, PLA-108, and PLA-117. Presumed Zika samples used in Figures 1 to 6: PARS_64, PARS_55, PARS_71, PARS_70, and PARS_97.

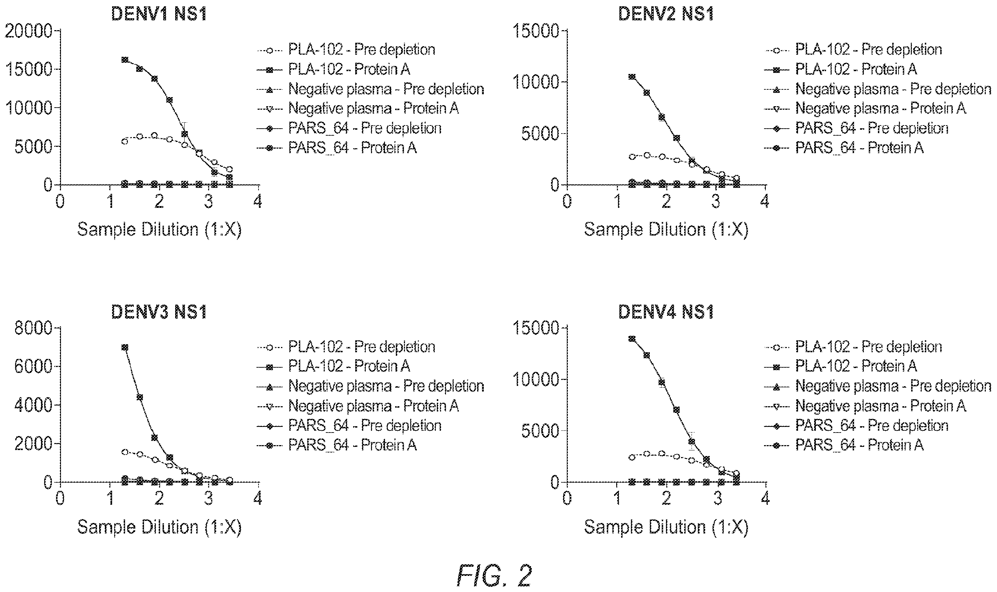

**Figure 2** Determination of the IgG3 amount in different patient samples with the method according to the present invention (designated as Protein A) and a method which did not include the Protein A depletion step before the actual determination of IgG3 (designated as pre-depletion). DENV1 NS1 protein (upper left panel), DENV2 NS1 protein (upper right panel), DENV3 NS1 protein (lower left panel), and DENV4 NS1 protein (lower right panel), respectively, have been used as antigen.

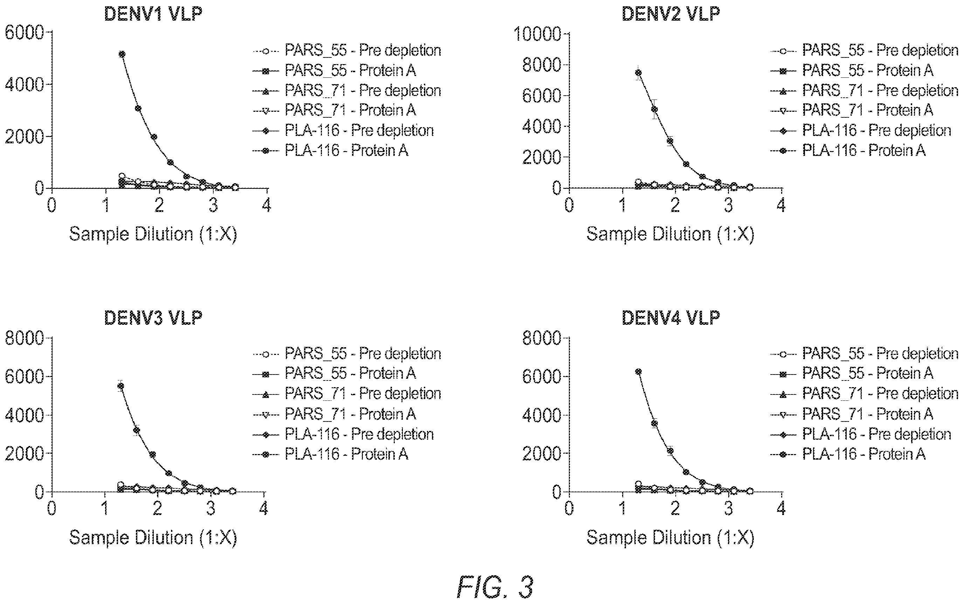

**Figure 3** Determination of the IgG3 amount in different patient samples with the method according to the present invention (designated as Protein A) and a method which did not include the Protein A depletion step before the actual determination of IgG3 (designated as pre-depletion). DENV1 VLP (upper left panel), DENV2 VLP (upper right panel), DENV3 VLP (lower left panel) and DENV4 VLP (lower right panel), respectively, have been used as antigen.

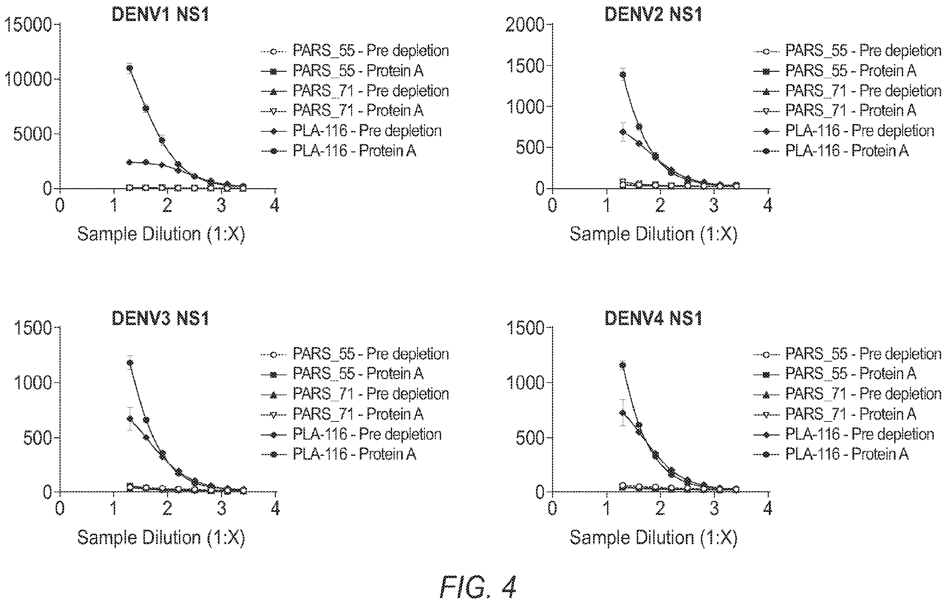

**Figure 4** Determination of the IgG3 amount in different patient samples with the method according to the present invention (designated as Protein A) and a method which did not include the Protein A depletion step before the actual determination of IgG3 (designated as pre-depletion). DENV1 NS1 protein (upper left panel), DENV2 NS1 protein (upper right panel), DENV3 NS1 protein (lower left panel), and DENV4 NS1 protein (lower right panel), respectively, have been used as antigen.

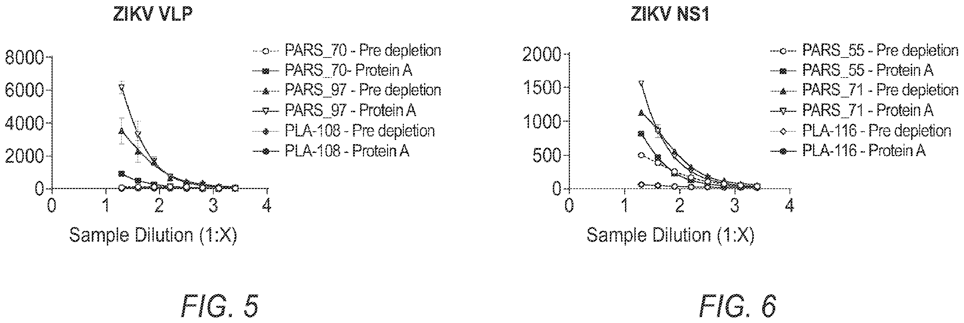

**Figure 5** Determination of the IgG3 amount in different patient samples with the method according to the present invention (designated as Protein A) and a method which did not include the Protein A depletion step before the actual determination of IgG3 (designated as pre-depletion). Zika VLP has been used as antigen.
**Figure 6** Determination of the IgG3 amount in different patient samples with the method according to the present invention (designated as Protein A) and a method which did not include the Protein A depletion step before the actual determination of IgG3 (designated as pre-depletion). Zika NS1 protein has been used as antigen.

### Detailed Description of the Invention

In a first aspect the present invention provides a method for determining the presence and/or amount of a human IgG3 antibody specific for a flavivirus antigen in a sample comprising the steps of:
**Step 1:** contacting an amount of the sample with Protein A coupled to beads to allow binding of human IgG1, human IgG2, human IgG4, human IgA, human IgE and human IgM antibodies to the Protein A coupled to the beads;
**Step 2:** separating the human IgG1, human IgG2, human IgG4, human IgA, human IgE and human IgM antibodies bound to the Protein A coupled to the beads from the remaining sample and thereby producing a human IgG3 antibody enriched supernatant;
**Step 3:** removing the human IgG3 antibody enriched supernatant from the human IgG1, human IgG2, human IgG4, human IgA, human IgE and human IgM antibodies bound to the Protein A coupled to the beads;
**Step 4:** contacting a flavivirus antigen with the human IgG3 antibody enriched supernatant to allow binding of the human IgG3 antibody to the flavivirus antigen;
**Step 5:** contacting the human IgG3 antibody bound to the flavivirus antigen with a primary
   IgG specific antibody or a primary IgG3 specific antibody to allow binding of the primary IgG specific antibody or the primary IgG3 specific antibody to the human IgG3 antibody bound to the flavivirus antigen;
**Step 6:** contacting the bound primary IgG3 specific antibody or the bound primary IgG specific antibody of step 4 with a labelled secondary antibody being specific for the primary IgG3 specific antibody or the primary IgG specific antibody to allow binding of the labelled secondary antibody to the primary antibody; and
**Step 7:** detecting a signal from the labelled secondary antibody bound to the primary antibody in step 5, wherein the signal is indicative for the presence and/or amount of the labelled secondary antibody and wherein the presence and/or amount of the labelled secondary antibody is indicative for the presence and/or amount of human IgG3 antibody specific for a flavivirus antigen in the sample; or wherein Steps 5 to 7 are replaced by:
   **Step 5':** contacting the human IgG3 antibody bound to the flavivirus antigen with a labelled primary IgG specific antibody or a labelled primary IgG3 specific antibody to allow binding of the labelled primary IgG specific antibody or the labelled primary IgG3 specific antibody to the human IgG3 antibody bound to the flavivirus antigen; and
   **Step 6':** detecting a signal from the labelled primary IgG specific antibody or a labelled primary IgG3 specific antibody bound to the flavivirus antigen in step 5', wherein the signal is indicative for the presence and/or amount of the labelled primary IgG specific antibody or a labelled primary IgG3 specific antibody and wherein the presence and/or amount of the labelled primary antibody is indicative for the presence and/or amount of human IgG3 antibody specific for a flavivirus antigen in the sample.

Step 1 of the method according to the invention comprises: contacting an amount of the sample with Protein A coupled to beads to allow binding of human IgG1, human IgG2, human IgG4, human IgA, human IgE and human IgM antibodies to the Protein A coupled to the beads.

"Protein A" herein includes Protein A as such. Protein A is a 49 kDa surface protein originally found in the cell wall of the bacterium *Staphylococcus aureus.* It is encoded by the *spa* gene and its regulation is controlled by DNA topology, cellular osmolarity, and a two-component system called ArlS-ArlR. It has found use in biochemical research because of its ability to bind immunoglobulins. The term "protein A" herein also includes sequence variants of Protein A, which differ by the addition, substitution and/or deletion of at least one amino acid, preferably not more than 10 amino acids, more preferably not more than 5 amino acids and most preferably by not more than one amino acids. The term also includes conjugates of Protein A with peptides or proteins.

Protein A coupled to beads can be obtained commercially. Alternatively, it can be prepared by coupling of Protein A to beads. The beads are commercially available. The material of the beads may be sepharose or sephadex. The coupling may be effected by using activated moieties on the surface of the beads. Alternatively, magnetic beads may be used. Protein A coupled to magnetic beads is commercially available (AmMag^{™} Protein A Magnetic beads; Genscript).

"Sample" herein includes blood samples. Preferably, the sample is serum from a human individual. The individual may be virus infected patient or a healthy individual immunized with a viral vaccine. The virus isa RNA virus being a flavivirus. Even more preferably, the RNA virus is dengue virus, Yellow fever virus, Japanese encephalitis virus, Tick-Borne encephalitis virus, West Nile virus, Zika virus, HIV virus, influenza virus, rotavirus, RSV, coronavirus, measles, mumps or rabies. Most preferred the RNA virus is dengue virus, Yellow fever virus, West Nile virus, Zika virus and coronavirus. In particular, the RNA virus is dengue virus or a Zika virus. If the RNA virus is dengue virus, the virus may be selected from DENV1, DENV2, DENV3 and/or DENV4.

The vaccine used for vaccination of the individual may preferably be a dengue vaccine, e.g. a tetravalent vaccine such as TAK-003 (Takeda).

The contacting step can be performed in plate format or in tube format. Alternatively, it could be performed in a column format. Preferably, plates with 24 or 96 wells are used. In a first step the plates are incubated with the viral antigen to allow binding of human antibodies other than human IgG3 to the Protein A-coupled beads.

Conditions for binding of the human IgG1, human IgG2, human IgG4, human IgA, human IgE and human IgM to Protein A coupled to the beads are known to the skilled person. Strong acidic or basic conditions should be avoided in order to prevent denaturation of Protein A and/or the human antibodies. Preferably, the protein A loading takes place at a pH in the range from about pH 8.0 to about pH 10.0. More preferably, the loading takes place at pH 9.0 in 1 M potassium phosphate.

Step 2 of the method according to the invention comprises: separating the human IgG1, human IgG2, human IgG4, human IgA, human IgE and human IgM antibodies bound to the Protein A coupled to the beads from the remaining sample and thereby producing a human IgG3 antibody enriched supernatant.

The separation of the antibody bound Protein A being coupled to the beads from the remaining sample can be effected by centrifugation. If the beads are magnetic beads, the separation may be effected by magnetic force. This can be accomplished e.g. by using a magnetic stand.

Step 3 of the method according to the invention comprises: removing the human IgG3 antibody enriched supernatant from the human IgG1, human IgG2, human IgG4, human IgA, human IgE and human IgM antibodies bound to the Protein A coupled to the beads.

The remaining sample depleted of human IgG1, human IgG2, human IgG4, human IgA, human IgE and human IgM is then removed from the beads. This may be effected by aspiration or decanting.

Step 4 of the method according to the invention comprises: contacting a flavivirus antigen with the human IgG3 antibody enriched supernatant to allow binding of the human IgG3 antibody to the virus antigen.

A "virus antigen" herein refers to any substance which can be bound by an Ab. Antigens may induce an immune response within a subject. An antigen may have one or more epitopes. An antigen may be a protein, polypeptide, carbohydrate, polynucleotide, lipid, or combinations thereof. An antigen may be a truncated version of a protein, a protein tagged with an affinity tag such as a His- or a STREP-tag, or a single domain of a protein. As used herein, antigen may e.g. refer to a DENV1 VLP, DENV2 VLP, DENV3 VLP, DENV4 VLP, ZIKV VLP; ZIKV NS1, DENV1 NS1, DENV2 NS1, DENV3 NS1, DENV4 NS1, DENV1 E, DENV2 E, DENV3 E and/or DENV4 E. Consequently, the term flavivirus antigen refers to an antigen from a flavivirus.

The terms "virus like particle (VLP)" or "virus like particles (VLPs)" refer to molecules that closely resemble viruses, but are non-infectious because they do not contain viral genetic material. VLPs can be prepared recombinant through the expression of viral structural proteins, which can then self-assemble into the VLPs. Examples of VLPs are ZIKV VLPs and DENV VLPs.

The contacting step can be performed in plate format. Preferably, 48 well or 96 well plates may be used. In a first step the plates are incubated with the viral antigen to allow binding of the viral antigen to the surface of the plate. If different viral antigens e.g. from different flaviviruses such as dengue or Zika, or antigens from different dengue virus serotypes are to be tested, the antigens may be in different wells (singleplex format) or in the same well (multiplex format). Methods for carrying out singleplex and multiplex assays are known to the skilled person.

The contacting step may be followed by a blocking step known to the person skilled in the art. The conditions for incubation and blocking must be such that the viral antigen does not significantly denature. Any of these steps may be followed by a washing step.

Step 5 of the method according to the invention comprises: contacting the human IgG3 antibody bound to the flavivirus antigen with a primary IgG specific antibody or a primary IgG3 specific antibody to allow binding of the primary IgG specific antibody or the primary IgG3 specific antibody to the human IgG3 antibody bound to the flavivirus antigen.

The primary IgG specific antibody is an anti-IgG antibody capable of specifically binding to more than one IgG subtype. Preferably, the primary IgG specific antibody is a pan-IgG antibody specifically binding to each of the IgG subtypes, i.e. IgG1, IgG2, IgG3 and IgG4. Pan-IgG antibodies are commercially available.

Alternatively, a primary IgG3 specific antibody may be used. Such antibodies are commercially available as well or can be produced in a manner known in the art. Preferably, the primary IgG specific antibody and the primary IgG3 specific antibody is of non-human origin. More preferably, a mouse, rat, hamster of goat antibody may be used.

In a separate embodiment the primary IgG specific antibody or the primary IgG3 specific antibody is coupled to a detectable label. Accordingly, in this embodiment Steps 5 to 7 as defined above are replaced by
**Step 5':** contacting the human IgG3 antibody bound to the virus antigen with a labelled primary IgG specific antibody or a labelled primary IgG3 specific antibody to allow binding of the labelled primary IgG specific antibody or the labelled primary IgG3 specific antibody to the human IgG3 antibody bound to the flavivirus antigen; and
**Step 6':** detecting a signal from the labelled primary IgG specific antibody or a labelled primary IgG3 specific antibody bound to the virus antigen in step 5', wherein the signal is indicative for the presence and/or amount of the labelled primary IgG specific antibody or a labelled primary IgG3 specific antibody and wherein the presence and/or amount of the labelled primary antibody is indicative for the presence and/or amount of human IgG3 antibody specific for a virus antigen in the sample.

Step 6 of the method according to the invention comprises: contacting the bound primary IgG3 specific antibody or the bound primary IgG specific antibody of step 4 with a labelled secondary antibody being specific for the primary IgG3 specific antibody or the primary IgG specific antibody to allow binding of the labelled secondary antibody to the primary antibody.

If the primary IgG specific antibody or the primary IgG3 specific antibody is not labelled, the secondary antibody being specific for the primary IgG specific antibody or the primary IgG3 specific antibody is labelled.

The term "label", as used herein, refers to any compound or moiety that comprises one or more appropriate chemical substances or enzymes, which directly or indirectly generate a detectable compound or signal in a chemical, physical or enzymatic reaction. Labeling can be achieved by methods well known in the art (see, for example, Lottspeich, F., and Zorbas H., Springer Spektrum 2012, Bioanalytik).

As used herein, the term "labelled antibody", refers to an Ab that is connected to a detectable label. The connection can be a covalent connection, which occurs for instance upon formation of an amide bound between the antibody and the detectable label. The type of connection is dependent on the functional groups available on the Ab and the label. In preferred embodiments, the antibody is attached to the label with the heavy chain constant region of the antibody.

The label may be selected from a chemiluminescent, fluorescent or an enzyme label. The enzyme may be horseradish peroxidase, alkaline oxidase, glucose oxidase or alkaline phosphatase. The substrate for the alkaline phosphatase may be adamantyl 1 or 2-dioxetane aryl phosphate (AMPPD) and for horseradish peroxidase the substrate may be luminol or its derivatives as a substrate.

In a preferred embodiment the label may be a fluorescence label selected from the group consisting of xanthene, fluorescein isothiocyanate, rhodamine, phycoerythrin, cyanine, coumarin, and any derivative thereof.

In a further preferred embodiment the label may be a chemiluminescent label selected from acridinium and ruthenium esters.

Step 7 of the method according to the invention comprises: detecting a signal from the labelled secondary antibody bound to the primary antibody in step 5, wherein the signal is indicative for the presence and/or amount of the labelled secondary antibody and wherein the presence and/or amount of the labelled secondary antibody is indicative for the presence and/or amount of human IgG3 antibody specific for the flavirus antigen in the sample.

The signal from the labelled secondary antibody is detected using a detection system. Any system which is suitable for determining values indicative for the presence and/or amount of the labelled secondary antibody bound to the bound primary IgG specific or primary IgG3 specific antibody may be used. The detection system comprises one or more light sources. Detection systems are commercially available and well known to the skilled person.

In a further aspect the present invention provides the use of a pre-treatment of a sample containing human IgG3 and one or more of human IgG1, human IgG2, human IgG4, human IgA and human IgM antibodies with immobilized or immobilizable Protein A *in vitro* for increasing the sensitivity of a method for determining the presence and/or amount of the human IgG3 antibody specific for a flavivirus antigen in the sample. The pre-treatment step may be carried out as described above for steps 1 to 3. Protein A may be immobilized by a covalent or a non-covalent bond to a solid support. Preferably, Protein A may be coupled to beads as outlined above. The sample may be a blood sample from a human subject. Preferably, the sample may contain human serum. The sample may further contain water, buffer salts and/or protein stabilizers.

In a further aspect the present invention provides a method for determining the human IgG3 response to a flaviviral vaccine, preferably a dengue or Zika vaccine comprising performing the method according to the invention. The vaccine used for vaccination of the individual may preferably be a dengue or Zika vaccine, e.g. a tetravalent dengue vaccine such as TAK-003 (Takeda). In a preferred embodiment the human IgG3 response to at least one, preferably each of the different dengue serotypes is determined. The dengue serotypes include DENV1, DENV2, DENV3 and DENV4.

In a further aspect the present invention provides a method for the *in vitro* diagnosis of a flavivirus infection in a human subject within the last six months, preferably a dengue virus or a Zika virus infection, comprising performing the method according to the invention, preferably a dengue virus or Zika virus infection. In a preferred embodiment the human IgG3 response to at least one, preferably, each of the different dengue serotypes is determined. The dengue serotypes include DENV1, DENV2, DENV3 and DENV4.

Dengue virus infections and Zika virus infections result mostly in asymptomatic symptoms and as such can be difficult, without laboratory confirmation, making accurate estimates of the disease incidence. Direct confirmation of infection using tools such as PCR and detection of NS1 antigen have limited time windows (within the first few days after the onset of symptoms) for maximum sensitivity. In contrast, anti-dengue NS1-specific IgG3 is detected very early after the onset of symptoms and has a wider detection window (4 to 6 months).

In a separate preferred embodiment the human subject is infected by at least two different flaviviruses. Preferably, the human subject is infected by a dengue virus and a Zika virus. The infection may be acute or convalescent. For instance, the subject has been infected by DENV first and was then infected by ZIKV several months later. The in vitro method for diagnosing of the present application is capable of diagnosing the at least two different flavivirus infections e.g. the DENV and the ZIKV infection. Consequently, the in vitro method for diagnosing of the present application is capable of determining whether a subject was infected with one or more flaviviruses and by which flaviviruses the subject was infected. This can be very useful in practice as multiple infections with different flaviviruses are commonly observed as flaviviruses are co-circulating in several areas.

Alternatively, the human subject is infected by at least two different dengue virus serotypes. For instance, the subject can be infected by DENV serotype 1 and DENV serotype 2. The infection may be acute or convalescent. For instance, the subject has been infected by DENV serotype 1 first and was then infected by DENV serotype 2 several months later. The in vitro method for diagnosing of the present application is capable of diagnosing the at least two different dengue virus serotype infections e.g. the DENV serotype 1 and the DENV serotype 2 infection. Consequently, the *in vitro* method for diagnosing of the present application is capable of determining whether a subject was infected with one or more DENV serotypes and by which DENV serotypes the subject was infected.

### EXAMPLES

### Materials and Methods

Orbital Plate Shaker Heidolph set was used. Protein A beads - AmMag^{™} Protein A Magnetic Beads were commercially obtained (Genscript; Cat. No. L00695). The magnetic plate was obtained from Life Technology (Cat. No. 032513). The Benchtop centrifuge was obtained from Thermo Scientific (Tag# 305395; S/N 42204931). The microplates were obtained from Costar (Ref.No. 39155). The Dynamag magnetic stand for 2 mL and 50mL tubes was obtained from Invitrogen (Cat. Nos. 12321D and 12302D, respectively).

### Samples

As samples, presumed samples have been obtained commercially. Dengue samples: PLA-102, PLA-116, PLA-108, PLA-117; and as Zika samples: PARS_64, PARS_55, PARS_71, PARS_70, PARS_97. Samples were purchased from the company ABO Pharmaceuticals.

### Preparation of the magnetic Beads

Before use the magnetic beads were resuspended and the resulting slurry transferred to a 50 mL conical tube. The magnetic beads were washed twice using a binding/wash buffer DPBS (1x) (Gibco). Then, the magnetic beads were washed twice with 0.1 N NaOH. Thereafter, the washing in binding/wash buffer as described above, was repeated twice.

### Depletion of human antibodies other than human IgG3 from the samples

The bead suspension pre-treated as described above was resuspended. 120µl of the suspension are provided to each of the wells of a 96-well plate according to a pre-determined plate layout. The plate was put on a magnetic plate for at least 30 seconds and then flicked to remove the supernatant. 70µl of sample were applied to the wells of the microplate according to the pre-determined plate layout and mixed with a pipet. The plates were incubated with mixing on a plate shaker (600 rpm) overnight (16-20 hours) at 37°C. The plates were spun down at 1000 rpm for one minute at 4°C. The suspension was transferred to 0.5 mL tube and a magnetic stand was used to separate the beads from the samples. The supernatant was carefully removed from the suspension in the plates. The supernatant was then further analyzed for the presence of IgG3 as described below.

### Detection of anti-Dengue NS1-specific IgG3 antibodies and anti-Zika NS1 specific IgG3 antibodies by ELISA

Half area 96-well plates (Coming, USA) were coated overnight, at 4"C, with either ZIKV NS1 (for ZIKV NS1 IgG3 assay, at equimolar ratio of all DENV serotypes (for DENV NS 1 IgG3 assay; Native Antigen, UK) at 2.27ug/mL. Plates were then blocked for 15 minutes at room temperature (RT). Serum samples and assay controls were diluted at 1:50 in blocking/dilution buffer, added to the wells in duplicate, and allowed to incubate for 1hr at RT. Sera from a recent ZIKV infection, which were collected 20-30 days post onset of symptoms, was used as ZIKV positive control. Pooled sera from recent dengue infection, which were collected 20-30 days post onset of symptoms, was used as DENV positive control. Pooled sera from healthy individuals naive to both ZIKV and DENV was used as negative control on both ZIKV and DENV assays. After washing 5 times, horseradish peroxidase (HRP)-conjugated mouse monoclonal antibody anti human IgG3 (Invitrogen, USA) was added to wells and incubated for 1hr at RT. After another washing cycle, TMB (KPL, USA) was added to the wells and allowed to incubate for 30 minutes at RT. Reaction was stopped with IN HCl (Sigma, USA) and optical densities were determined at wavelength of 450nm (OD450) using SpectraMax Plus PC380 microplate spectrophotometer and SoftMax Pro software version 6.4 (Molecular Devices, USA). OD450 of sample and control wells were subtracted from the average of OD450 of the conjugate blank wells before analysis.

For DENV IgG3 analysis, DENV ratio was determined by dividing the OD450 of the samples by the average OD450nm of the negative control. For ZIKV IgG3 analysis, ZIKV ratio was determined by dividing the OD450 of the samples by the average OD450 of the DENV recent infection.

The cut-off values for the DENV IgG3 and ZIKV IgG3 ratios were 1.5 and 0.586, respectively. A sample was defined as eligible for analysis if the average ratio of a sample was above the cut-off value (antigen-specific IgG3 positive sample) and coefficient of variation [(Standard deviation of replicates/average of replicates) x l00] of replicates was below 20%.

### Detection of anti-Dengue VLP-specific IgG3 antibodies and anti-Zika VLP specific IgG3 antibodies by ELISA

The same procedure as outlined above was followed with the difference that Dengue VLP and Zika VLP, respectively, as antigen, instead of the NS1 antigen described above.

### Results

Figure 1 shows a comparison of the results which have been obtained with the method according to the present invention (designated as Protein A) with a method which did not include the Protein A depletion step before the actual determination of IgG3 (designated as pre-depletion). It can be seen that for each of the four different DENV VLPs as antigen the method according to the invention resulted in a significantly increased sensitivity compared to the method without the prior Protein A depletion step.

Figure 2 shows a comparison of the results which have been obtained with the method according to the present invention (designated as Protein A) with a method which did not include the Protein A depletion step before the actual determination of IgG3 (designated as pre-depletion). It can be seen that for each of the four different DENV NS1 proteins as antigen the method according to the invention resulted in a significantly increased sensitivity compared to the method without the prior Protein A depletion step.

Figure 3 relates to a comparison of the results which have been obtained with the method according to the present invention (designated as Protein A) with a method which did not include the Protein A depletion step before the actual determination of IgG3 (designated as pre-depletion). Figure 3 differs from Figure 1 in that different patient samples have been tested, but the same four different DENV VLPs have been used as antigens. Also here, the method according to the invention resulted in a significantly increased sensitivity.

Figure 4 shows a comparison of the results which have been obtained with the method according to the present invention (designated as Protein A) with a method which did not include the Protein A depletion step before the actual determination of IgG3 (designated as pre-depletion). Figure 4 differs from Figure 1 in that different patient samples have been tested, but the same four different DENV NS1 proteins as antigen have been used as antigens. The method according to the invention also provided a significantly increased sensitivity.

Figure 5 shows the application of the present method to IgG3 antibodies specific for Zika Virus VLP. It could be demonstrated that the method according to the invention also resulted in an increased sensitivity for such antibodies.

Figure 6 shows that the present method could be successfully applied to IgG3 antibodies specific for Zika NS1 protein. The method according to the invention resulted in increased sensitivity compared to a method lacking the prior depletion of antibodies other than IgG3 from the sample.

## Claims

1. A method for determining the presence and/or amount of a human IgG3 antibody specific for a flavivirus antigen in a sample comprising the steps of:
**Step 1:** contacting an amount of the sample with Protein A coupled to beads to allow binding of human IgG1, human IgG2, human IgG4, human IgA, human IgE and human IgM antibodies to the Protein A coupled to the beads;
**Step 2:** separating the human IgG1, human IgG2, human IgG4, human IgA, human IgE and human IgM antibodies bound to the Protein A coupled to the beads from the remaining sample and thereby producing a human IgG3 antibody enriched supernatant;
**Step 3:** removing the human IgG3 antibody enriched supernatant from the human IgG1, human IgG2, human IgG4, human IgA, human IgE and human IgM antibodies bound to the Protein A coupled to the beads;
**Step 4:** contacting a flavivirus antigen with the human IgG3 antibody enriched supernatant to allow binding of the human IgG3 antibody to the flavivirus antigen;
**Step 5:** contacting the human IgG3 antibody bound to the flavivirus antigen with a
primary IgG specific antibody or a primary IgG3 specific antibody to allow binding of the primary IgG specific antibody or the primary IgG3 specific antibody to the human IgG3 antibody bound to the flavivirus antigen;
**Step 6:** contacting the bound primary IgG3 specific antibody or the bound primary IgG specific antibody of step 4 with a labelled secondary antibody being specific for the primary IgG3 specific antibody or the primary IgG specific antibody to allow binding of the labelled secondary antibody to the primary antibody; and
**Step 7:** detecting a signal from the labelled secondary antibody bound to the primary antibody in step 5, wherein the signal is indicative for the presence and/or amount of the labelled secondary antibody and wherein the presence and/or amount of the labelled secondary antibody is indicative for the presence and/or amount of human IgG3 antibody specific for a flavivirus antigen in the sample;
or wherein Steps 5 to 7 are replaced by:
**Step 5':** contacting the human IgG3 antibody bound to the flavivirus antigen with a
labelled primary IgG specific antibody or a labelled primary IgG3 specific antibody to allow binding of the labelled primary IgG specific antibody or the labelled primary IgG3 specific antibody to the human IgG3 antibody bound to the flavivirus antigen; and
**Step 6':** detecting a signal from the labelled primary IgG specific antibody or a labelled primary IgG3 specific antibody bound to the flavivirus antigen in step 5', wherein the signal is indicative for the presence and/or amount of the labelled primary IgG specific antibody or a labelled primary IgG3 specific antibody and wherein the presence and/or amount of the labelled primary antibody is indicative for the presence and/or amount of human IgG3 antibody specific for a flavivirus antigen in the sample.

2. The method of claim 1, wherein the IgG3 antibody is specific for a dengue virus antigen or a Zika virus antigen.

3. The method according to any one of claims 1 to 2, wherein the antigen is selected from the group consisting of virus like particle (VLP), non-structural protein 1, envelope protein, pre-membrane protein, membrane protein, capsid protein, non-structural protein 2A, non-structural protein 2B, non-structural protein 3, non-structural protein 4A, non-structural protein 4B, and non-structural protein 5 and any derivative thereof, preferably the antigen is VLP.

4. The method according to any one of claims 1 to 3, wherein step 1 is carried out in a plate or tube format, preferably in 96 well microplates.

5. The method according to any one of claims 1 to 4, wherein the protein A is coupled to magnetic beads and the separating step 2 is performed by applying magnetic force.

6. The method according to any one of claims 1 to 5, wherein the protein A is coupled to beads and the separating step 2 is performed by gravitational force, preferably by applying a step of centrifugation.

7. The method according to any one of claims 1 to 6, wherein the human IgG3 antibody is enriched in the IgG3 supernatant compared to the content in the starting sample by a factor of at least 2, preferably at least 5, more preferably at least 10.

8. The method according to claim 1, wherein the label of the secondary antibody in step 6 includes a chemiluminescent, fluorescent or an enzyme label.

9. The method according to claim 1, wherein the label of the primary antibody in step 5' includes a chemiluminescent, fluorescent or an enzyme label.

10. The method according to claim 8 or 9, wherein the enzyme is horseradish peroxidase, alkaline oxidase or glucose oxidase.

11. The method according to claim 10, wherein the label is a fluorescence label selected from the group consisting of xanthene, fluorescein isothiocyanate, rhodamine, phycoerythrin, cyanine, coumarin, and any derivative thereof.

12. The method according to any one of claims 1 and 2 to 11, wherein steps 4 to 6 are performed in the format of a sandwich assay.

13. The method according to any one of claims 1 and 2 to 12, wherein steps 4 to 7 are performed in microplate format, preferably in the format of 96 well plates.

14. The method according to claim 1, wherein steps 4, 5' and 6' are performed in microplate format, preferably in the format of 96 well plates.

15. A method for increasing the sensitivity of a method for determining the presence and/or amount of the human IgG3 antibody specific for a flavivirus antigen in a sample comprising performing a step of pre-treatment of the sample containing human IgG3 specific for a flavivirus antigen and one or more of human IgG1, human IgG2, human IgG4, human IgA and human IgM antibodies with immobilized or immobilizable Protein A.

16. The method according to claim 15, wherein the Protein A is coupled to beads.

17. The method according to claim 15 or 16, wherein the sample is a blood sample from a human subject, more preferably the sample is from human serum.

18. A method for determining the human IgG3 response to a flaviviral vaccine, preferably a dengue or Zika vaccine comprising performing the method according to any one of claims 1 to 14.

19. The method according to claim 18, wherein the human IgG3 response to at least one dengue serotypes selected from DENV1, DENV2, DENV3 and DENV4, preferably to each of the different dengue serotypes is determined.

20. A method for the *in vitro* diagnosis of a flavivirus infection in a human subject within the last six months, preferably a dengue virus or a Zika virus infection, comprising performing the method according to any one of claims 1 to 14.

21. The method according to claim 20, wherein the flavivirus infection is an infection by at least one dengue serotype selected from DENV1, DENV2, DENV3 and DENV4, or a Zika virus infection.

22. The method according to claim 16, wherein the human subject is infected by at least two different flaviviruses, preferably dengue virus and Zika virus.

## Patentansprüche

1. Verfahren zum Bestimmen der Anwesenheit und/oder Menge eines menschlichen IgG3-Antikörpers spezifisch für ein Flavivirusantigen in einer Probe, umfassend die folgenden Schritte:
**Schritt 1:** Kontaktieren einer Menge der Probe mit Protein A, das an Kügelchen gekoppelt ist, um ein Binden eines menschlichen IgG1-, menschlichen IgG2-, menschlichen IgG4-, menschlichen IgA-, menschlichen IgE- und menschlichen IgM-Antikörpers an das an die Kügelchen gekoppelte Protein A zu ermöglichen;
**Schritt 2:** Trennen des menschlichen IgG1-, menschlichen IgG2-, menschlichen IgG4-, menschlichen IgA-, menschlichen IgE- und menschlichen IgM-Antikörpers, die an das an die Kügelchen gekoppelte Protein A gebunden sind, von der verbleibenden Probe und dadurch Herstellen eines mit einem menschlichen IgG3-Antikörper angereicherten Überstands;
**Schritt 3:** Entfernen des mit menschlichen IgG3-Antikörpern angereicherten Überstands aus dem menschlichen IgG1-, menschlichen IgG2-, menschlichen IgG4-, menschlichen IgA-, menschlichen IgE- und menschlichen IgM-Antikörper, die an das an die Kügelchen gekoppelte Protein A gebunden sind;
**Schritt 4:** Kontaktieren eines Flavivirusantigens mit dem mit einem menschlichen IgG3-Antikörper angereicherten Überstand, um ein Binden des menschlichen IgG3-Antikörpers an das Flavivirusantigen zu ermöglichen;
**Schritt 5:** Kontaktieren des an das Flavivirusantigen gebundenen menschlichen IgG3-Antikörpers mit einem primären IgG-spezifischen Antikörper oder einem primären IgG3-spezifischen Antikörper, um ein Binden des primären IgG-spezifischen Antikörpers oder des primären IgG3-spezifischen Antikörpers an den an das Flavivirusantigen gebundenen menschlichen IgG3-Antikörper zu ermöglichen;
**Schritt 6:** Kontaktieren des gebundenen primären IgG3-spezifischen Antikörpers oder des gebundenen primären IgG-spezifischen Antikörpers von Schritt 4 mit einem markierten sekundären Antikörper, der spezifisch für den primären IgG3-spezifischen Antikörper oder den primären IgG-spezifischen Antikörper ist, um ein Binden des markierten sekundären Antikörpers an den primären Antikörper zu ermöglichen; und
**Schritt 7:** Detektieren eines Signals von dem markierten sekundären Antikörper, der an den primären Antikörper von Schritt 5 gebunden ist, wobei das Signal die Anwesenheit und/oder die Menge des markierten sekundären Antikörpers anzeigt und wobei die Anwesenheit und/oder Menge des markierten sekundären Antikörpers die Anwesenheit und/oder Menge eines menschlichen IgG3-Antikörpers anzeigt, der spezifisch für ein Flavivirusantigen in der Probe ist; oder wobei die Schritte 5 bis 7 durch Folgendes ersetzt werden:
**Schritt 5':** Kontaktieren des an das Flavivirusantigen gebundenen menschlichen IgG3-Antikörpers mit einem markierten primären IgG-spezifischen Antikörper oder einem markierten primären IgG3-spezifischen Antikörper, um ein Binden des markierten primären IgG-spezifischen Antikörpers oder des markierten primären IgG3-spezifischen Antikörpers an den an das Flavivirusantigen gebundenen menschlichen IgG3-Antikörper zu ermöglichen; und
**Schritt 6':** Detektieren eines Signals von dem markierten primären IgG-spezifischen Antikörper oder einem markierten primären IgG3-spezifischen Antikörper, der an das Flavivirusantigen in Schritt 5' gebunden ist, wobei das Signal die Anwesenheit und/oder Menge des markierten primären IgG-spezifischen Antikörpers oder eines markierten primären IgG3-spezifischen Antikörpers anzeigt und wobei die Anwesenheit und/oder Menge des markierten primären Antikörpers die Anwesenheit und/oder Menge eines menschlichen IgG3-Antikörpers anzeigt, der spezifisch für ein Flavivirusantigen in der Probe ist.

2. Verfahren nach Anspruch 1, wobei der IgG3-Antikörper spezifisch für ein Denguevirusantigen oder ein Zikavirusantigen ist.

3. Verfahren nach einem der Ansprüche 1 bis 2, wobei das Antigen aus der Gruppe ausgewählt ist, bestehend aus virusartigen Partikeln (VLP), nicht-strukturellem Protein 1, Hüllprotein, Prä-Membranprotein, Membranprotein, Kapsidprotein, nicht-strukturellem Protein 2A, nicht-strukturellem Protein 2B, nicht-strukturellem Protein 3, nicht-strukturellem Protein 4A, nicht-strukturellem Protein 4B und nicht-strukturellem Protein 5 und einem beliebigen Derivat davon, wobei das Antigen bevorzugt VLP ist.

4. Verfahren nach einem der Ansprüche 1 bis 3, wobei Schritt 1 in einem Platten- oder Röhrchenformat, bevorzugt in 96-Well-Mikrotiterplatten, durchgeführt wird.

5. Verfahren nach einem der Ansprüche 1 bis 4, wobei das Protein A an magnetische Kügelchen gekoppelt ist und der Trennschritt 2 durch Anwenden von Magnetkraft durchgeführt wird.

6. Verfahren nach einem der Ansprüche 1 bis 5, wobei das Protein A an Kügelchen gekoppelt ist und der Trennschritt 2 durch Schwerkraft, bevorzugt durch Anwenden eines Zentrifugationsschritts, durchgeführt wird.

7. Verfahren nach einem der Ansprüche 1 bis 6, wobei der menschliche IgG3-Antikörper in dem IgG3-Überstand verglichen mit dem Inhalt in der Ausgangsprobe um einen Faktor von mindestens 2, bevorzugt mindestens 5, mehr bevorzugt mindestens 10, angereichert ist.

8. Verfahren nach Anspruch 1, wobei die Markierung des sekundären Antikörpers in Schritt 6 eine chemilumineszente, fluoreszierende oder eine Enzymmarkierung beinhaltet.

9. Verfahren nach Anspruch 1, wobei die Markierung des primären Antikörpers in Schritt 5' eine chemilumineszente, fluoreszierende oder eine Enzymmarkierung beinhaltet.

10. Verfahren nach Anspruch 8 oder 9, wobei das Enzym Meerrettichperoxidase, alkalische Oxidase oder Glucoseoxidase ist.

11. Verfahren nach Anspruch 10, wobei die Markierung eine Fluoreszenzmarkierung ist, die aus der Gruppe ausgewählt ist, bestehend aus Xanthen, Fluoresceinisothiocyanat, Rhodamin, Phycoerythrin, Cyanin, Cumarin und einem beliebigen Derivat davon.

12. Verfahren nach einem der Ansprüche 1 und 2 bis 11, wobei die Schritte 4 bis 6 in dem Format eines Sandwich-Assays durchgeführt werden.

13. Verfahren nach einem der Ansprüche 1 und 2 bis 12, wobei die Schritte 4 bis 7 in einem Mikrotiterplattenformat, bevorzugt in dem Format von 96-Well-Platten, durchgeführt werden.

14. Verfahren nach Anspruch 1, wobei die Schritte 4, 5' und 6' in einem Mikrotiterplattenformat, bevorzugt in dem Format von 96-Well-Platten, durchgeführt werden.

15. Verfahren zur Erhöhen der Empfindlichkeit eines Verfahrens zum Bestimmen der Anwesenheit und/oder Menge des menschlichen IgG3-Antikörpers spezifisch für ein Flavivirusantigen in einer Probe, umfassend ein Durchführen eines Schritts einer Vorbehandlung der Probe, die für ein Flavivirusantigen spezifisches menschliches IgG3 und einen oder mehrere von einem menschlichen IgG1-, menschlichen IgG2-, menschlichen IgG4-, menschlichen IgA- und menschlichen IgM-Antikörper enthält, mit immobilisiertem oder immobilisierbarem Protein A.

16. Verfahren nach Anspruch 15, wobei das Protein A an Kügelchen gekoppelt ist.

17. Verfahren nach Anspruch 15 oder 16, wobei die Probe eine Blutprobe von einem menschlichen Subjekt ist, wobei die Probe mehr bevorzugt von menschlichem Serum ist.

18. Verfahren zur Bestimmen der menschlichen IgG3-Reaktion auf einen Flavivirusimpfstoff, bevorzugt einen Dengue- oder Zikaimpfstoff, umfassend das Durchführen des Verfahrens nach einem der Ansprüche 1 bis 14.

19. Verfahren nach Anspruch 18, wobei die menschliche 1gG3-Reaktion auf mindestens einen Dengueserotypen, ausgewählt aus DENV1, DENV2, DENV3 und DENV4, bevorzugt auf jeden der verschiedenen Dengueserotypen, bestimmt wird.

20. Verfahren zur In-vitro-Diagnose einer Flavivirusinfektion bei einem menschlichen Subjekt innerhalb der letzten sechs Monate, bevorzugt einer Denguevirus- oder einer Zikavirusinfektion, umfassend das Durchführen des Verfahrens nach einem der Ansprüche 1 bis 14.

21. Verfahren nach Anspruch 20, wobei die Flavivirusinfektion eine Infektion durch mindestens einen Dengueserotyp, ausgewählt aus DENV1, DENV2, DENV3 und DENV4, oder eine Zikavirusinfektion ist.

22. Verfahren nach Anspruch 16, wobei das menschliche Subjekt mit mindestens zwei verschiedenen Flaviviren, bevorzugt Denguevirus und Zikavirus, infiziert ist.

## Revendications

1. Procédé pour déterminer la présence et/ou la quantité d'un anticorps humain IgG3 spécifique d'un antigène de flavivirus dans un échantillon, comprenant les étapes suivantes :
**Étape 1** : mise en contact d'une quantité de l'échantillon avec la protéine A couplée à des billes pour permettre la liaison des anticorps humains IgG1, IgG2, IgG4, IgA, IgE et IgM à la protéine A couplée aux billes ;
**Étape 2** : séparation des anticorps humains IgG1, IgG2, IgG4, IgA, IgE et IgM liés à la protéine A couplée aux billes de l'échantillon restant et production ainsi d'un surnageant enrichi en anticorps humain IgG3 ;
**Étape 3 :** retrait du surnageant enrichi en anticorps humain IgG3 des anticorps humains IgG1, IgG2, IgG4, IgA, IgE et IgM liés à la protéine A couplée aux billes ;
**Étape 4** : mise en contact d'un antigène de flavivirus avec le surnageant enrichi en anticorps humain IgG3 pour permettre la liaison de l'anticorps humain IgG3 à l'antigène de flavivirus ;
**Étape 5** : mise en contact de l'anticorps humain IgG3 lié à l'antigène de flavivirus avec un anticorps primaire spécifique IgG ou un anticorps primaire spécifique IgG3 pour permettre la liaison de l'anticorps primaire spécifique IgG ou de l'anticorps primaire spécifique IgG3 à l'anticorps humain IgG3 lié à l'antigène de flavivirus ;
**Étape 6** : mise en contact de l'anticorps primaire spécifique IgG3 lié ou l'anticorps primaire spécifique IgG lié à l'étape 4 avec un anticorps secondaire marqué étant spécifique de l'anticorps primaire spécifique IgG3 ou de l'anticorps primaire spécifique IgG pour permettre la liaison de l'anticorps secondaire marqué à l'anticorps primaire ; et
**Étape 7:** détection d'un signal provenant de l'anticorps secondaire marqué lié à l'anticorps primaire à l'étape 5, dans lequel le signal est indicatif de la présence et/ou de la quantité de l'anticorps secondaire marqué et dans lequel la présence et/ou la quantité de l'anticorps secondaire marqué sont indicatives de la présence et/ou de la quantité d'anticorps humain IgG3 spécifiques d'un antigène de flavivirus dans l'échantillon ;
ou dans lequel les étapes 5 à 7 sont remplacées par :
**Étape 5'** : mise en contact de l'anticorps humain IgG3 lié à l'antigène de flavivirus avec un anticorps primaire spécifique IgG marqué ou un anticorps primaire spécifique IgG3 marqué pour permettre la liaison de l'anticorps primaire spécifique IgG marqué ou de l'anticorps primaire spécifique IgG3 marqué à l'anticorps humain IgG3 lié à l'antigène de flavivirus ; et
**Étape 6'** : détection d'un signal provenant de l'anticorps primaire spécifique IgG marqué ou d'un anticorps primaire spécifique IgG3 marqué lié à l'antigène de flavivirus à l'étape 5', dans lequel le signal est indicatif de la présence et/ou de la quantité de l'anticorps primaire spécifique IgG marqué ou d'un anticorps primaire spécifique IgG3 marqué, et dans lequel la présence et/ou la quantité de l'anticorps primaire marqué sont indicatives de la présence et/ou de la quantité d'anticorps humain IgG3 spécifiques d'un antigène de flavivirus dans l'échantillon.

2. Procédé selon la revendication 1, dans lequel l'anticorps IgG3 est spécifique d'un antigène du virus de la dengue ou d'un antigène du virus Zika.

3. Procédé selon l'une quelconque des revendications 1 à 2, dans lequel l'antigène est choisi parmi le groupe composé de particules de type viral (VLP), de protéines non structurales 1, de protéines d'enveloppe, de protéines pré-membranaires, de protéines membranaires, de protéines de capside, de protéines non structurales 2A, 2B, 3, 4A, 4B et 5 et de tout dérivé de celles-ci, de préférence l'antigène est une VLP.

4. Procédé selon l'une quelconque des revendications 1 à 3, dans lequel l'étape 1 est réalisée dans un format de plaque ou de tube, de préférence dans des microplaques à 96 puits.

5. Procédé selon l'une quelconque des revendications 1 à 4, dans lequel la protéine A est couplée à des billes magnétiques et l'étape 2 de séparation est réalisée en appliquant une force magnétique.

6. Procédé selon l'une quelconque des revendications 1 à 5, dans lequel la protéine A est couplée à des billes et l'étape 2 de séparation est réalisée par la force gravitationnelle, de préférence en appliquant une étape de centrifugation.

7. Procédé selon l'une quelconque des revendications 1 à 6, dans lequel l'anticorps humain IgG3 est enrichi dans le surnageant IgG3 par rapport à la teneur dans l'échantillon de départ d'un facteur d'au moins 2, de préférence d'au moins 5, plus particulièrement d'au moins 10.

8. Procédé selon la revendication 1, dans lequel le marquage de l'anticorps secondaire à l'étape 6 comporte un marquage chimioluminescent, fluorescent ou enzymatique.

9. Procédé selon la revendication 1, dans lequel le marquage de l'anticorps primaire à l'étape 5' comporte un marquage chimioluminescent, fluorescent ou enzymatique.

10. Procédé selon la revendication 8 ou 9, dans lequel l'enzyme est la peroxydase de raifort, l'oxydase alcaline ou la glucose oxydase.

11. Procédé selon la revendication 10, dans lequel le marquage est un marquage fluorescent choisi parmi le groupe composé de xanthène, d'isothiocyanate de fluorescéine, de rhodamine, de phycoérythrine, de cyanine, de coumarine et de tout dérivé de ceux-ci.

12. Procédé selon l'une quelconque des revendications 1 et 2 à 11, dans lequel les étapes 4 à 6 sont réalisées sous la forme d'un dosage sandwich.

13. Procédé selon l'une quelconque des revendications 1 et 2 à 12, dans lequel les étapes 4 à 7 sont réalisées sous forme de microplaques, de préférence sous forme de plaques à 96 puits.

14. Procédé selon la revendication 1, dans lequel les étapes 4, 5' et 6' sont réalisées sous forme de microplaques, de préférence sous forme de plaques à 96 puits.

15. Procédé pour accroître la sensibilité d'un procédé pour déterminer la présence et/ou la quantité d'anticorps humain IgG3 spécifiques d'un antigène de flavivirus dans un échantillon, comprenant une étape de prétraitement de l'échantillon contenant des IgG3 humains spécifiques d'un antigène de flavivirus et un ou plusieurs des anticorps humains IgG1, IgG2, IgG4, IgA et IgM avec une protéine A immobilisée ou immobilisable.

16. Procédé selon la revendication 15, dans lequel la protéine A est couplée à des billes.

17. Procédé selon la revendication 15 ou 16, dans lequel l'échantillon est un échantillon de sang provenant d'un sujet humain, de préférence l'échantillon provient de sérum humain.

18. Procédé pour déterminer la réponse IgG3 humaine à un vaccin flaviviral, de préférence un vaccin contre la dengue ou le Zika, comprenant la réalisation du procédé selon l'une quelconque des revendications 1 à 14.

19. Procédé selon la revendication 18, dans lequel la réponse IgG3 humaine à au moins un sérotype de dengue choisi parmi DENV1, DENV2, DENV3 et DENV4, de préférence à chacun des différents sérotypes de dengue, est déterminée.

20. Procédé de diagnostic *in vitro* d'une infection par le flavivirus chez un sujet humain au cours des six derniers mois, de préférence une infection par le virus de la dengue ou le virus Zika, comprenant la réalisation du procédé selon l'une quelconque des revendications 1 à 14.

21. Procédé selon la revendication 20, dans lequel l'infection par le flavivirus est une infection par au moins un sérotype de dengue choisi parmi DENV1, DENV2, DENV3 et DENV4, ou une infection par le virus Zika.

22. Procédé selon la revendication 16, dans lequel le sujet humain est infecté par au moins deux flavivirus différents, de préférence le virus de la dengue et le virus Zika.
